# FASCICULE DE BREVET EUROPEEN

(11) **EP 2 659 274 B1**
(45) Date de publication et mention de la délivrance du brevet: **24.06.2015**
(21) Numéro de dépôt: 11802102.1
(22) Date de dépôt: 22.12.2011
(51) Int. Cl.: A61B 5/00, A61D 7/00, A61K 49/00, G01N 33/58, A01K 45/00, C12N 15/89, G01N 33/68

(54) **Procédé de contrôle d'une injection d'une composition luminescente dans un oeuf aviaire à des fins vaccinales**
Verfahren zur Kontrolle der Injektion einer leuchtenden-Komposition-Impfstoff im Ei
Method of controlling an injection of a luminescent composition in an avian egg for vaccine purposes

(30) Priorité: 27.12.2010 FR 1061278
(43) Date de publication de la demande: 06.11.2013
(73) Titulaire: Ceva Sante Animale, 33500 Libourne Cedex (FR)
(72) Inventeur: MASSONNEAU, Marc, F-27570 Tilliers sur Avre (FR); MOGENET, Laurent, F-33500 Libourne (FR)
(74) Mandataire: Vidon Brevets & Stratégie
(86) Numéro de dépôt international: PCT/EP2011/073869
(87) Numéro de publication internationale: WO 2012/089631

(56) Documents cités:
- EP-A1- 1 127 486
- WO-A1-00/08924
- WO-A1-2009/042723
- WO-A1-2010/103111
- WO-A2-00/55339
- WO-A2-2006/078499
- WO-A2-2010/008562
- US-A- 4 458 630
- US-A- 5 028 421
- RAAB U ET AL: "Endoglin is expressed in the chicken vasculature and is involved in angiogenesis", FEBS LETTERS, ELSEVIER, AMSTERDAM, NL, vol. 459, no. 2, 8 octobre 1999 (1999-10-08), pages 249-254, XP004260356, ISSN: 0014-5793, DOI: DOI:10.1016/S0014-5793(99)01252-1

## Description

### 1. Domaine technique de l'invention

L'invention se rapporte à l'utilisation de composés aptes à émettre un signal luminescent détectable dans un oeuf aviaire.

L'invention concerne particulièrement l'utilisation de ces biomarqueurs dans un procédé de détection et/ou de quantification de phénomène biologique à des fins vaccinales et/ou thérapeutiques et/ou diagnostiques dans un oeuf aviaire.

L'invention est plus particulièrement destinée au domaine de la vaccination *in ovo.*

### 2. Arrière-plan technologique

L'imagerie optique gagne en popularité dans le domaine biomédical avec nombres d'applications précliniques et humaines dans des domaines tels que la recherche sur le cancer, pour la détection de tumeurs ou encore en neurosciences, pour l'imagerie du cerveau.

Ainsi, comme cela a été décrit dans le document de brevet publié sous le numéro WO2008/025006 sur des souris ou des petits animaux, des photons émis par la dispersion des cellules marquées par un composé luminescent excité par une lumière diffusent à travers les tissus de ces mammifères. Même si nombres de ces photons diffus sont absorbés, une fraction atteint la surface où ils peuvent être détectés par une caméra qui enregistre en deux dimensions (2D) la distribution spatiale des photons émis par la surface.

Toutefois, même dans le cas d'une sonde fluorescente unique dans une souris, l'autofluorescence tissulaire rend difficile la bonne résolution.

Afin de tenter de palier ces déficiences, deux axes principaux ont été explorés :
- adaptation des dispositifs d'imagerie optiques selon les orientations décrites dans le document de brevet publié sous le numéro WO2007 144542 ;
- adaptation des sondes luminescentes : nombres de sondes ont été développées mais l'attention de la demanderesse a porté plus particulièrement sur les biomarqueurs, tels que ceux décrits dans le document de brevet publié sous le numéro WO2006/129036. Cependant, ces informations ne permettent pas et ne suggèrent pas d'appliquer l'imagerie optique sur des oeufs.

WO 2010/103111 nous apprend que des anticorps injectable are marqués préférablement avec des composés luminescents, comme par exemple un groupement fluorescent ou phosphorescent. WO 2010/103111 a trait aux procédés de détermination du sexe des embryons. Ce procédé comprend notamment l'injection d'un anticorps marqué, lequel se fixera spécifiquement à certains antigènes de l'embryon.

US 4 458 630 divulgue un procédé de vaccination in ovo, notamment par injection de souches virales spécifiques. US 5 028 421 divulgue également un procédé de vaccination in ovo, notamment par injection d'interleukine aviaire.

### 3. Objectifs de l'invention

L'une des applications privilégiée de l'invention est appliquée à la vaccination *in ovo.*

Un certain nombre de techniques ont été élaborées jusqu'à présent pour permettre la vaccination *in ovo* d'embryons, c'est-à-dire la vaccination d'embryons alors que ces derniers sont encore dans l'oeuf. Il est en effet reconnu que ces vaccinations *in ovo* permettent une diminution des coûts, une forte automatisation de la vaccination, une réduction du stress et augmente le taux de succès par rapport à une vaccination des poussins après éclosion.

L'une des difficultés de cette opération de vaccination *in ovo* réside dans le contrôle de la vaccination, la taille de l'oeuf pouvant varier de façon significative. En effet, un oeuf fécondé comprend une pluralité de compartiments distincts, dont la coquille externe, la chambre à air, le liquide allantoïde, le liquide amniotique et l'embryon. Or, une vaccination n'est efficace que si le produit est injecté dans l'embryon ou le liquide amniotique.

Il est en pratique difficile de certifier que l'injection a été proprement réalisée, notamment sur les chaînes automatisées de traitement des oeufs aviaires, tels que les chaînes de traitement des oeufs des futurs poulets de chair.

L'une des solutions couramment utilisées de nos jours consiste à mélanger le produit injecté avec un colorant et à échantillonner de large quantité d'oeufs sur la chaîne de vaccination pour vérifier la qualité de l'injection. Si l'oeuf ainsi testé est jugé non conforme, alors le dispositif d'injection est ré-étalonné.

L'un des inconvénients de cette méthode est qu'elle nécessite la destruction de plusieurs centaines voir de milliers d'oeufs, donc d'embryons, sans néanmoins garantir une bonne reproductibilité de la vaccination et nécessite une mise en place lourde en terme de personnel et de temps. Un autre inconvénient lié à cette destruction massive d'oeufs est l'impossibilité d'appliquer cette méthode en routine et le caractère invasif qui est non compatible avec les standards d'automatisation, de productivité et de bien-être.

Il existe aussi un besoin pour une méthode de contrôle pour pouvoir garantir que la vaccination et/ou le prélèvement sont effectués dans les conditions optimales.

Dès lors, l'invention vise à pallier au moins certains des inconvénients des procédés et dispositifs de l'état de la technique.

En particulier, l'invention vise à fournir un procédé de contrôle de la qualité d'une injection de produit dans un oeuf qui soit non invasif.

L'invention vise aussi à fournir un tel procédé dont la fiabilité n'est pas seulement liée à un calibre d'oeuf.

L'invention vise aussi à proposer un dispositif mettant en oeuvre un procédé selon l'invention.

### 4. Exposé de l'invention

L'objet de la présente demande est limité par l'objet des revendications 1-17. La demande concerne toute composition luminescente injectable dans un oeuf caractérisée en ce qu'elle comprend un composé apte à émettre un signal luminescent (ou fluorescent ou autoluminescent ou chimiluminescent) détectable dans un oeuf formant biomarqueur d'un produit ayant une activité vaccinale, thérapeutique ou diagnostique.

On notera que les composés luminescents selon la demande utilisés pour marquer un produit ayant une telle activité vaccinale, se distinguent clairement des anticorps marqués détectables par une excitation lumineuse connus de la demande WO2010/103111 et mis en oeuvre pour déterminer *in ovo* le sexe des espèces aviaires.

On entend par composé apte à émettre un signal luminescent, tout composé qui émet une radiation lors de son passage d'un état stable à un état excité ou vice-versa.

Selon un mode de réalisation particulier, ledit composé luminescent est le vert d'indocyanine qui émettra des photons suite à la soumission de l'oeuf à une source d'excitation lumineuse.

Selon une variante, la composition utilisée dans le procédé comprend en outre un produit ayant une activité vaccinale.

L'invention concerne un procédé de contrôle d'une injection d'un produit à des fins vaccinales dans un oeuf, comprenant successivement les étapes suivantes :
- injection d'une composition comprenant un produit ayant une activité vaccinale et un composé luminescent formant biomarqueur dudit produit ayant une activité vaccinale dans un oeuf ;
- détection du signal émis par le composé luminescent au travers de la coquille de l'oeuf ;
- traitement de l'information recueillie.

Ainsi, il ressort clairement qu'un procédé de contrôle selon l'invention est non invasif.

De plus, comme cela va apparaître plus clairement par la suite, la fiabilité d'un procédé selon l'invention est indépendante du calibre de l'oeuf analysé.

En outre, il est possible d'intégrer un procédé selon l'invention sur une chaîne automatique de vaccination par exemple.

L'invention réside donc dans l'utilisation d'un composé luminescent comme marqueur du produit injecté dans l'oeuf. La déposante a en effet découvert qu'il est possible de détecter les photons émis par le composé luminescent, ceci malgré la présence d'une barrière minérale, à savoir la coquille de l'oeuf.

Selon une solution avantageuse, le procédé comprend une étape de corrélation du signal émis par le composé luminescent et de la localisation de celui-ci dans l'oeuf.

On peut de cette façon obtenir une indication claire et directe de la position du produit injecté dans l'oeuf, et vérifier si cette position est adaptée ou pas par rapport à l'effet escompté du produit injecté.

Selon une solution avantageuse, le procédé comprend une étape de soumission de l'oeuf à une source d'excitation lumineuse nécessaire à la détection du signal. Le composé luminescent est ainsi excité par la source lumineuse adaptée qui peut être préférentiellement réalisée à l'aide d'un laser ou d'une source d'excitation lumineuse appropriée comprenant, notamment, les filtres adaptés aux longueurs d'ondes du luminophore.

Selon une variante avantageuse, ladite étape de soumission de l'oeuf à une source d'excitation lumineuse et ladite étape de détection du signal sont effectuées de part et d'autre de l'axe de symétrie dudit oeuf.

Avantageusement, lesdites étapes de soumission et de traitement de l'information recueillie sont effectuées pour au moins deux, préférentiellement au moins trois, positions angulaires distinctes relatives entre l'oeuf et ladite source d'excitation lumineuse.

Ces positions angulaires distinctes pourront être obtenues tandis que l'oeuf est mis en rotation sur lui-même. On pourra aussi envisager d'obtenir ces positions en maintenant l'oeuf fixe et en faisant varier la position de la source d'émission lumineuse et/ou la position des moyens servant au traitement de l'information recueillie.

On peut de cette façon obtenir un ensemble de signaux représentatifs d'une « image » en trois dimensions de la position du composé luminescent et donc de celle du produit injecté, dans l'oeuf.

Les signaux obtenus selon les différentes positions pourront aussi faire l'objet d'une sélection de façon à ne retenir que le meilleur signal en vue de ladite étape de corrélation.

Selon une solution avantageuse, ladite étape de détection du signal est réalisée à l'aide d'une caméra numérique.

Egalement, selon une variante de l'invention, ladite étape de traitement de l'information recueillie n'est effectuée que sur la partie du signal émis par la partie supérieure de l'oeuf contenant la chambre à air.

Selon un mode de réalisation particulier, le procédé comprend une étape préalable de modélisation du signal émis par le composé luminescent lorsqu'il se situe dans chacune des zones suivantes de l'oeuf :
- chambre à air ;
- liquide allantoïde ;
- liquide amniotique ;
- embryon.

Dans ce cas, ladite étape d'analyse est préférentiellement réalisée à l'aide d'une unité de traitement dans laquelle sont mémorisés des signaux modélisés obtenus lors de ladite étape préalable.

Dans ce cas, le procédé comprend avantageusement une étape de comparaison du signal émis par le composé luminescent au travers de la coquille de l'oeuf pendant l'étape d'analyse avec les signaux modélisés obtenus lors de ladite étape préalable.

Selon une première approche de l'invention, ladite étape d'injection est conduite de façon à injecter simultanément ledit produit à injecter et ledit composé luminescent.

Selon une deuxième approche de l'invention, ladite étape d'injection est conduite de façon à injecter séparément ledit produit à injecter et ledit composé luminescent.

Dans ce cas, ledit composé luminescent est apte à venir se fixer sur le produit.

Dans l'un ou l'autre cas, on note que le composé luminescent est prévu pour occuper une position dans l'oeuf confondue avec celle du produit injecté.

Selon un mode de réalisation particulier, ledit composé luminescent est le vert d'indocyanine (CAS n°3599-32-4) de formule générale C₄₃H₄₇N₂NₐO₆S₂ :

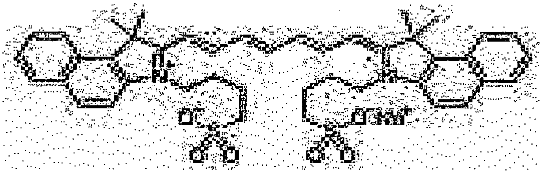

Préférentiellement, ce composé luminescent est un composé de formule générale de type : S-B-A ou ses dérivés, tels que des sels, des esters ou des dérivés fonctionnalisés avec des éléments structuraux décrits et revendiqués par le document de brevet publié sous le numéro FR-2 886 292.

Un procédé selon l'invention peut être utilisé pour contrôler et/ou quantifier dans un oeuf :
- un vaccin;

La demande concerne également un dispositif pour la mise en oeuvre des étapes de soumission et d'analyse d'un procédé selon l'invention, caractérisé en ce qu'il comprend,
- une zone d'accueil d'au moins un oeuf ;
- optionnellement au moins une source d'excitation lumineuse dirigée vers ladite zone d'accueil ;
- des moyens de détection du signal émis par le composé luminescent au travers de la coquille de l'oeuf;
- des moyens de traitements de l'information recueillie.

Préférentiellement, ladite zone d'accueil est montée mobile en rotation.

Le dispositif selon l'invention pourra se présenter sous la forme d'un dispositif autonome et/ou d'un dispositif intégré dans une chaîne automatisée de traitement d'oeufs, telle qu'une chaîne automatisée de vaccination.

### 5. Liste des figures

D'autres buts, caractéristiques et avantages de l'invention apparaîtront à la lecture de la description suivante donnée à titre uniquement non limitatif et qui se réfère aux figures annexées, dans lesquelles :
- la figure 1 est une vue schématique d'un mode de réalisation d'un dispositif pour la mise en oeuvre d'un procédé selon l'invention ;
- les figures 2 à 5 sont des vues schématiques de différents signaux émis par un composé luminescent au cours d'un procédé selon l'invention ;
- la figure 6 est un ensemble de trois images correspondant à des prises de vue par une caméra numérique d'un oeuf soumis à une source d'excitation lumineuse (laser) et dans la chambre à air duquel un composé luminescent a été injecté ;
- la figure 7 est une image correspondant à une prise de vue par une caméra numérique d'un oeuf dans la chambre à air duquel un composé luminescent a été injecté et à laquelle est associé un axe vertical Y sur lequel sont réparties N = 400 lignes horizontales;
- la figure 8 est un graphe traduisant la répartition de l'intensité lumineuse moyenne (Unité Arbitraire d'intensité relative) de l'image de la figure 7 selon l'axe vertical Y
- les figures 9 à 12 représentent les images et les graphes de répartition de l'intensité lumineuse selon l'axe vertical Y associés constituant des signaux modélisés pour des oeufs dans lesquels le composé luminescent a été injecté respectivement dans la chambre à air, le liquide allantoïde, le liquide amniotique et l'embryon ;
- la figure 13 représente un graphe de répartition de l'intensité lumineuse associé à un oeuf dans lequel un composé luminescent à été injecté;
- la figure 14 représente le même graphe que celui de la figure 13 mais dans lequel l'intégrale de la courbe portée sur celui-ci correspondant à l'intensité lumineuse de la partie de l'oeuf contenant la chambre à air (S1) et l'intégrale de la courbe portée sur celui-ci correspondant à l'intensité lumineuse du reste de l'oeuf (S1) ont été identifiées.

### 6. Description détaillée d'un mode de réalisation de l'invention

Dans toute la description ci-après, il est fait référence à l'utilisation d'un procédé selon l'invention pour contrôler l'injection d'un vaccin dans un oeuf.

Appliqué au contrôle de l'injection d'un vaccin, un procédé selon l'invention comprend les étapes suivantes :
- injection d'une composition comprenant un vaccin et un composé luminescent formant biomarqueur dudit vaccin dans un oeuf ;
- soumettre l'oeuf à une source d'excitation lumineuse ;
- détecter et analyser le signal émis par le composé luminescent au travers de la coquille de l'oeuf.

Dans le cadre du mode de réalisation ici décrit, le composé luminescent est le composé de formule C₄₃H₄₇N₂NₐO₆S₂ communément appelé le vert d'indocyanine (CAS n°3599-32-4) Une autre composé dénommé FR99, décrit et revendiqué par le document de brevet publié sous le numéro FR-2 886 292 a été évalué et des résultats non présentés confirme les observations faites avec le vert d'indocyanine. Ces composés sont chimiquement inertes vis-à-vis du vaccin. Les propriétés rhéologiques de la composition incluant le composé luminescent sont sensiblement les mêmes que celles du vaccin.

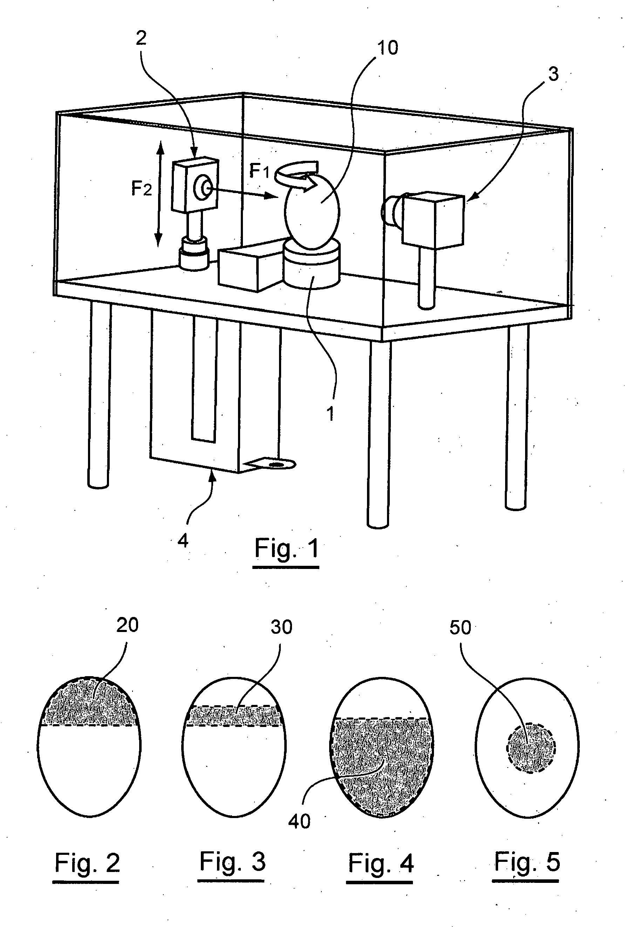

Un procédé selon la demande peut par exemple être mis en oeuvre avec un dispositif tel que celui illustré à la figure 1.

Tel que cela apparaît sur cette figure, un dispositif pour la mise en oeuvre d'un procédé de contrôle d'une injection de vaccin dans un oeuf comprend :
- une zone d'accueil 1 d'un oeuf 10 ;
- un laser 2, dont le faisceau est susceptible d'être dirigé vers la zone d'accueil, et plus précisément vers l'oeuf 10 ;
- le laser constituant une source d'excitation lumineuse du composé luminescent injecté dans l'oeuf ;
- une caméra numérique 3, constituant des moyens de détection du signal émis par le composé luminescent au travers de la coquille de l'oeuf ;
- une unité de traitement 4, destinée à recevoir et à traiter les données procurées par la caméra numérique 3.

Tel que représenté sur la figure 1, la zone d'accueil est placée entre le laser 2 et la caméra numérique 3. On note qu'il n'est pas nécessaire que le laser, la zone d'accueil et la caméra numérique soient alignés les uns par rapport aux autres.

La zone d'accueil 1 est montée mobile en rotation autour d'un axe vertical (tel qu'illustré par la flèche courbe F1), et est couplée à des moyens d'entraînement en rotation (non représentés).

Préalablement, à la phase de contrôle de la vaccination à l'aide d'un dispositif tel que décrit précédemment, on réalise une étape de modélisation du signal susceptible d'être émis par le composé luminescent injecté dans l'oeuf, sous excitation lumineuse, ceci pour chacune des zones suivantes de l'oeuf :
- la chambre à air ;
- le liquide allantoïde
- le liquide amniotique ;
- l'embryon.

Ainsi, lors de cette étape préalable de modélisation, le composé luminescent réagit à l'excitation lumineuse et procure, au travers de la coquille, en retour, un signal parmi ceux illustrés par les figures 2 à 5.

Ainsi, lorsque le composé luminescent se situe dans la chambre à air, on obtient une zone 20 lumineuse telle qu'illustrée par la figure 2, coïncidant sensiblement avec le volume de la chambre à air.

Lorsque le composé luminescent se situe dans le liquide allantoïde, on obtient une zone 30 lumineuse telle qu'illustrée par la figure 3, coïncidant sensiblement avec le volume du sac allantoïde.

Lorsque le composé luminescent se situe dans le liquide amniotique, on obtient une zone 40 lumineuse telle qu'illustrée par la figure 4, coïncidant sensiblement avec le volume occupé par le liquide amniotique.

Lorsque le composé luminescent se situe dans l'embryon, on obtient une zone 50 lumineuse, telle qu'illustrée par la figure 5, coïncidant sensiblement avec le volume occupé par l'embryon (qui bien sûr varie en fonction de son stade de développement).

Ces zones 20, 30, 40, 50 sont donc modélisées pour procurer des données sous forme informatique mémorisées par l'unité de traitement 4.

Lorsqu'un oeuf est soumis au procédé de contrôle selon l'invention à l'aide d'un dispositif tel que celui décrit précédemment, le vaccin et le composé luminescent sont injectés dans les oeufs.

Cette étape peut être conduite de telle sorte que l'injection du vaccin et l'injection du composé luminescent sont réalisées simultanément. Il s'en suit donc que le composé luminescent se situe dans la même zone que le vaccin, le vaccin et le composé luminescent étant injectés ensemble, voire mélangés.

Il est également possible d'injecter séparément le vaccin et le composé luminescent. Dans ce cas, le composé luminescent a une composition chimique prévue pour réagir à celle du vaccin de telle sorte que le composé luminescent vienne se placer dans la même zone de l'oeuf que le vaccin (ce qui peut également être le cas même si le composé luminescent est injecté simultanément avec le vaccin). Les compositions chimiques du composé luminescent et du vaccin peuvent être prévues de telle sorte que le composé luminescent se fixe sur le vaccin.

Les oeufs à contrôler sont donc soumis aux rayons du laser 2, l'analyse du signal émis par le composé luminescent au travers de la coquille de l'oeuf étant alors effectuée par la caméra numérique 3. L'image captée par la caméra numérique 3 est fournie sous forme de données numériques à l'unité de traitement 4 qui procède à une étape de corrélation du signal émis par le composé luminescent et de la localisation de celui-ci dans l'oeuf.

Pour cela, l'unité de traitement effectue une étape de comparaison du signal émis par le composé luminescent au travers de la coquille de l'oeuf, fourni par la caméra numérique, avec les signaux modélisés obtenus lors de l'étape préalable ayant conduit aux données mémorisées représentatives par exemple des figures 2 à 5.

Bien entendu, l'unité de traitement est paramétrée pour réaliser le contrôle sous forme d'un test : en fonction du produit injecté, il est attendu que l'injection de celui-ci soit réalisée dans une zone précise de l'oeuf et si les données représentatives de la position du produit injecté dans l'oeuf (ces données étant obtenues grâce au composé luminescent) ne correspondent pas aux données d'une position modélisée, le contrôle est négatif. Bien entendu, s'il y a correspondance entre les deux positions, le résultat est positif.

Pour augmenter la fiabilité du procédé selon l'invention, il est possible de soumettre l'oeuf à une excitation lumineuse et d'analyser le signal émis par le composé luminescent pour au moins deux positions angulaires de l'oeuf (autour de son axe vertical) par rapport aux rayons du laser. Préférentiellement, on réalise le contrôle tandis que la zone d'accueil 1 est mise en rotation (la zone d'accueil étant prévue pour que la rotation de la zone d'accueil entraîne celle de l'oeuf autour dé son axe vertical).

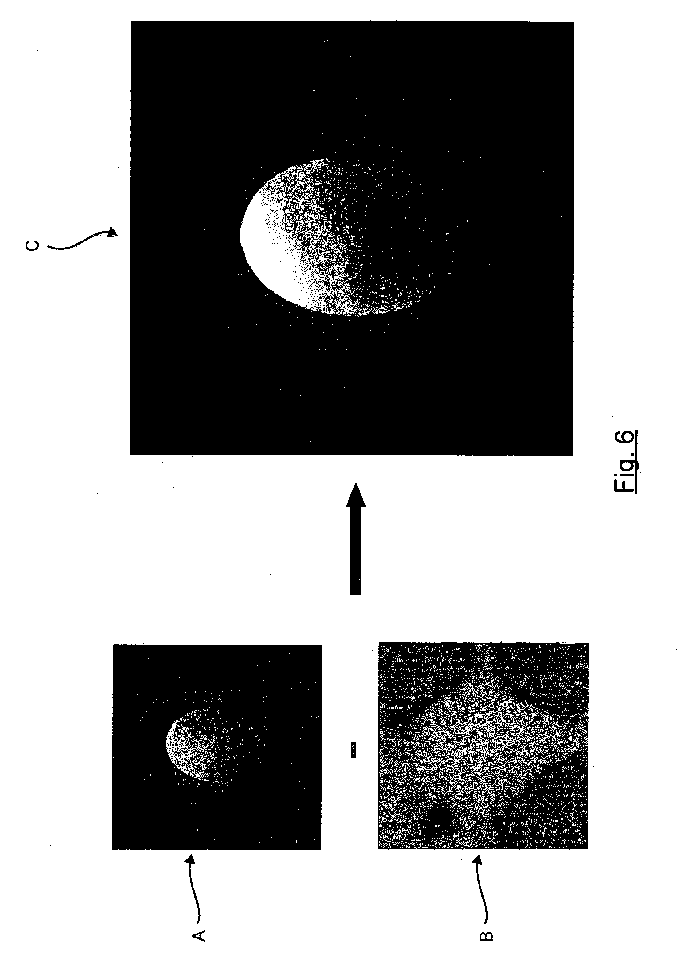

Le procédé selon l'invention a été testé de façon à mettre en évidence sa pertinence pour contrôler l'injection d'un vaccin. Les principaux résultats de ces différents tests effectués sont présentés ci-après en référence aux figures 6 à 14.

### Détermination de la concentration optimale de composé luminescent et de la durée optimale d'acquisition du signal

24 oeufs fécondés à 18,5 jours d'incubation ont fait l'objet d'une injection *in ovo* d'une solution de vert d'indocyanine à différentes concentrations, sensée correspondre à une solution vaccinale marquée par un marqueur luminescent. Différentes durées d'acquisition des signaux recueillis sous formes d'image par la caméra 3 ont été testées.

En tout, 720 images ont été analysées. Cette analyse a permis mettre en évidence que :
- la concentration optimale de composé luminescent (Cₒₚₜ) est de 50 micromoles par litre ;
- la durée optimale d'acquisition du signal (Tₒₚₜ) est de 90 secondes.

### Modélisation des signaux

Des tests ont ensuite été effectués pour modéliser des signaux émis par le composé luminescent lorsqu'il est injecté dans chacune des zones suivantes d'un oeuf :
- la chambre à air,
- le liquide allantoïde,
- le liquide amniotique,
- l'embryon.

A cet effet, des oeufs présentant des âges de fécondation différents, à savoir : 17 ; 17,5 ; 18 ; 18,5 et 19 jours d'incubation, ont fait l'objet de l'injection de vert d'indocyanine à la concentration optimale (Cₒₚₜ) déterminée ci-dessus.

Pour chaque âge de fécondation, l'injection a été effectuée à différentes profondeurs d'injection, à savoir : dans la chambre à air d'un oeuf, dans le liquide allantoïde d'un deuxième oeuf, dans le liquide amniotique d'un troisième oeuf et dans l'embryon d'un quatrième oeuf.

Pour chacun de ces oeufs, trois images ont été acquises, avant et après injection, par la caméra 3 pendant la durée d'acquisition optimale (Tₒₚₜ) selon trois positions relatives distinctes de l'oeuf par rapport à la caméra 3 et au rayon laser 2. Ces trois positions distinctes ont été obtenues en faisant tourner l'oeuf sur lui-même autour de son axe de symétrie de 120 ° puis de nouveau de 120 °.

Les images ainsi recueillies par la caméra 3 ont été ensuite été traitées par l'unité de traitement 4.

Ce traitement a d'abord consisté à traiter le signal en vue d'éliminer le bruit généré par la lumière cohérente traversant l'oeuf et ne résultant pas de la présence du vert d'indocyanine. En pratique, le signal obtenu après injection a été soustrait du signal obtenu avant injection, et ce pour chaque oeuf, pour chaque position et pour chaque profondeur d'injection.

La figure 6 montre l'image (A) obtenue pour un oeuf dans une position donnée avant injection du produit luminescent, l'image (B) obtenue pour le même oeuf dans la même position après injection du produit dans sa chambre à air à la concentration optimale définie ci-dessus, et, l'image traitée (C) obtenue par traitement des signaux correspondant aux images (A) et (B) ayant consisté à soustraire du signal correspondant à l'image (B) le signal correspondant à l'image (A).

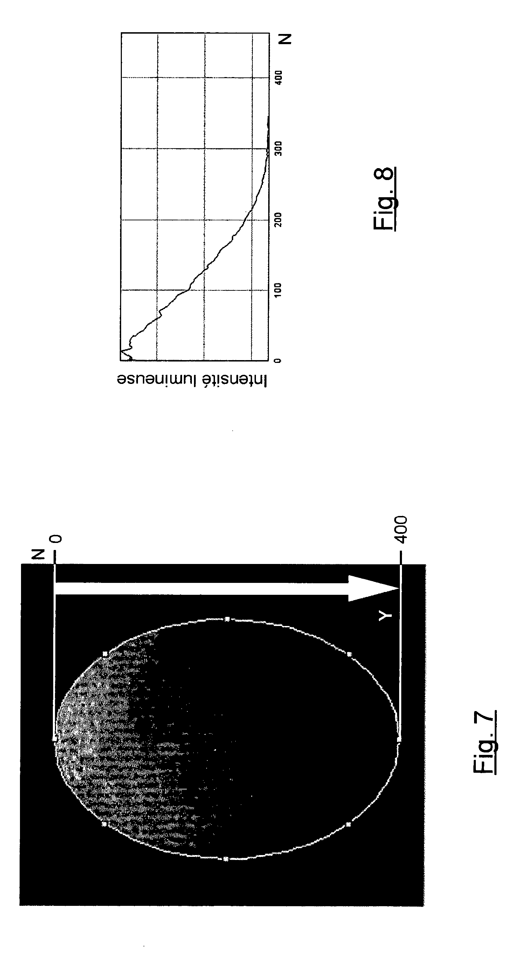

Pour chaque oeuf, les trois images traitées (C) obtenues pour les trois positions citées ci-dessus ont ensuite été analysées. En référence à la figure 7, cette analyse a consisté à effectuer pour chaque image une série de mesures de l'intensité lumineuse et à en faire une moyenne par ligne, de haut en bas de l'image selon l'axe Y, de façon à obtenir une répartition de l'intensité lumineuse moyenne selon cet axe telle qu'indiqué, pour un exemple, sur la figure 8. En pratique 400 mesures ont été effectuées par image (correspondant à 400 lignes horizontales des images) soit 1200 mesures par oeuf.

Pour chaque oeuf, parmi les trois images traitées et analysées, l'image' traitée montrant l'intensité lumineuse moyenne la plus élevée, traduisant au maximum la présence du produit fluorescent, a été sélectionnée.

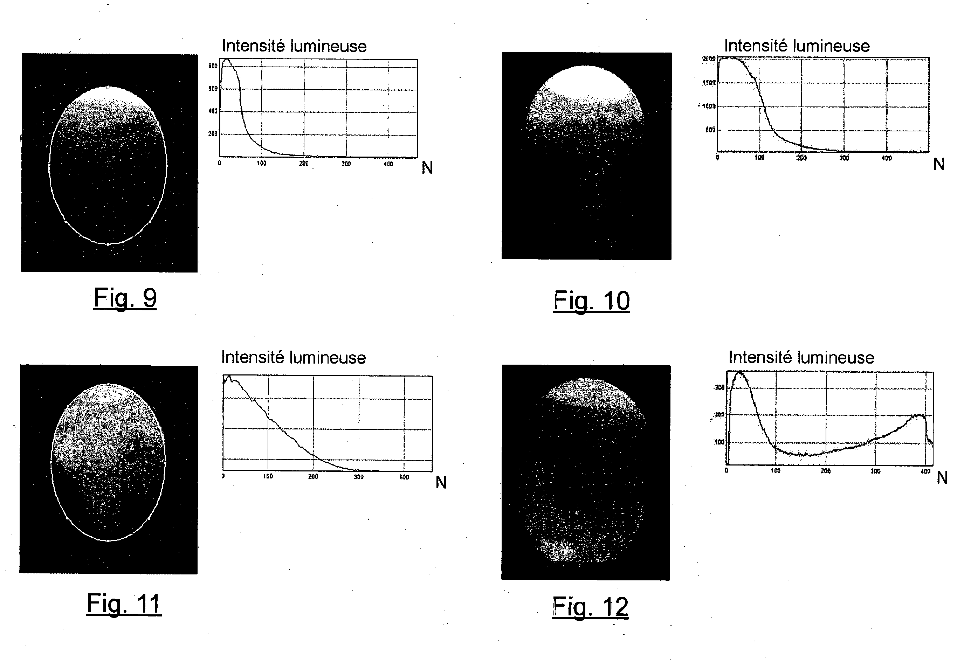

Ainsi, des modèles de signaux émis par le composé luminescent lorsqu'il a été injecté dans chacune des zones suivantes de l'oeuf : chambre à air, liquide allantoïde, liquide amniotique, embryon ont pu être obtenus. Ces signaux modélisés et les images correspondantes sont représentés aux figures 9, 10, 11 et 12 respectivement. Ils indiquent chacun l'intensité lumineuse moyenne relative (Unité Arbitraire d'intensité relative) (axe des ordonnées) relevée en fonction d'un nombre N de lignes, en l'occurrence 400, traduisant la hauteur de l'oeuf selon son axe vertical depuis son sommet (axe des abscisses).

Comme on peut le voir sur ces figures 9 à 12, chaque modèle présente un profil bien particulier. En référence à la figure 13, une intensité de luminescence globale moyenne peut de ce fait être associée à chaque compartiment de l'oeuf. Ainsi, le signal qui sera détecté et traité selon l'invention pour un oeuf ayant fait l'objet d'une injection d'un produit, tel que par exemple un vaccin, et de produit luminescent, pourra facilement être rapproché de l'un de ces modèles afin de déterminer dans quelle partie de l'oeuf en question l'injection a effectivement eu lieu.

A ce sujet, la forme de ces profils indique également qu'il pourra être envisagé de cantonner l'étape de comparaison à la partie du signal émise par la partie supérieure de l'oeuf contenant la chambre à air et donc ne de traiter que cette partie du signal en vue de ladite comparaison.

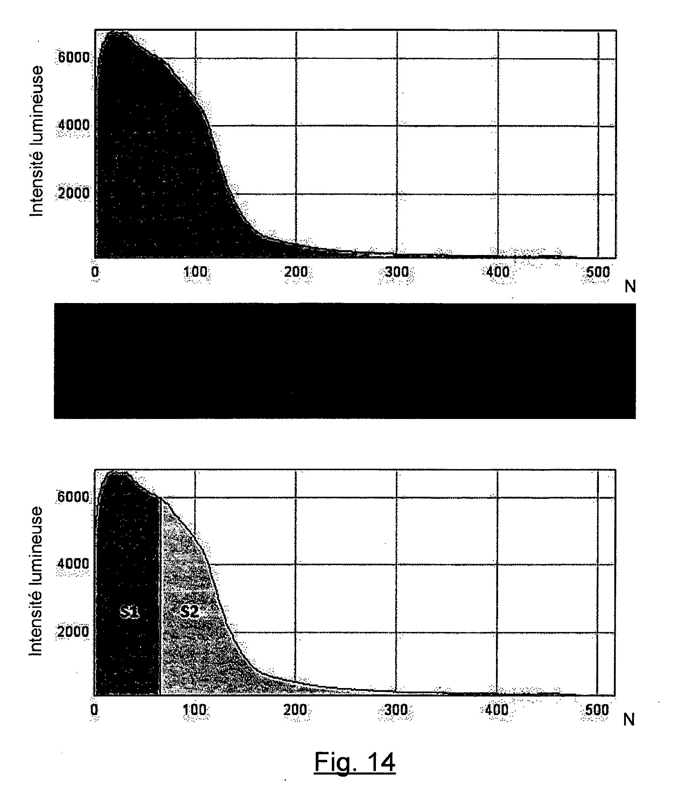

En référence à la figure 14, on note également qu'à chaque signal peut être associé un ratio S1/S2 traduisant la proportion de luminescence émise par la partie de l'oeuf correspondant sensiblement à celle contenant la chambre à air, par rapport à la luminescence émise par le reste de l'oeuf. Ainsi, en référence à la figure 15, un ratio de surface moyen peut également être associé à chaque compartiment de l'oeuf. Ce ratio pourra également être utilisé pour aider à la détermination de la partie de l'oeuf dans laquelle l'injection a effectivement eu lieu.

### Corrélation

Afin de valider la corrélation entre les images acquises et la localisation réelle de la solution injectée, un test a été effectué sur soixante oeufs présentant des âges de fécondation différents, à savoir: 17 ; 17,5,;18 ; 18,5 et 19 jours d'incubation (12 oeufs par âge).

Pour ce faire, 60 oeufs embryonnés de l'âge indiqué ont reçu une injection double (composé luminescent et teinture bleue) localisée de manière approximativement équilibrée entre les 4 compartiments. Puis un examen optique de l'oeuf sous les 3 angles suivi d'un décorticage complet de l'oeuf (permettant la caractérisation du positionnement de la teinture bleue) ont généré à la fois, et pour chaque oeuf, les données optiques d'intensité et de localisation précise de l'injection.

Ce test a permis de confirmer que les images acquises grâce au procédé selon l'invention étaient étroitement corrélées à la localisation réelle de la solution injectée dans l'oeuf.

## Revendications

1. Procédé de contrôle d'une injection d'un produit à des fins vaccinales dans un oeuf aviaire, comprenant successivement les étapes suivantes :
injection d'une composition comprenant un produit ayant une activité vaccinale et un composé luminescent formant biomarqueur dudit produit ayant une activité vaccinale dans un oeuf ;
- détection du signal émis par le composé luminescent au travers de la coquille de l'oeuf ;
- traitements de l'information recueillie.

2. Procédé selon la revendication 1, **caractérisé en ce que** ledit composé luminescent est un composé fluorescent ou autoluminescent ou chimiluminescent.

3. Procédé selon l'une quelconque des revendications précédentes, **caractérisé en ce que** ledit composé luminescent est le vert d'indocyanine ou ses dérivés, tels que des sels, des esters ou des dérivés fonctionnalisés avec des éléments structuraux.

4. Procédé selon l'une quelconque des revendications précédentes, **caractérisé en ce que** ledit composé luminescent est de type S-B-A ou ses dérivés, tels que des sels, des esters ou des dérivés fonctionnalisés avec des éléments structuraux.

5. Procédé selon l'une quelconque des revendications précédentes, **caractérisé en ce qu'**il comprend une étape de corrélation du signal émis par le composé luminescent et de la localisation de celui-ci dans l'oeuf.

6. Procédé selon l'une quelconque des revendications précédentes, **caractérisé en ce qu'**il comprend une étape de soumission de l'oeuf à une source d'excitation lumineuse nécessaire à la détection du signal.

7. Procédé selon la revendication 6, **caractérisé en ce que** la source d'excitation lumineuse est adaptée aux longueurs d'ondes du composé luminescent.

8. Procédé selon les revendications 6 ou 7, **caractérisé en ce que** la source d'excitation lumineuse est un laser ou tous filtres adaptés aux longueurs d'ondes du composé luminescent

9. Procédé selon l'une quelconque des revendications précédentes, **caractérisé en ce qu'**il comprend une étape préalable de modélisation du signal émis par le composé luminescent lorsqu'il se situe dans chacune des zones suivantes de l'oeuf :
- chambre à air,
- liquide allantoïde,
- liquide amniotique,
- embryon.

10. Procédé selon la revendication 9, **caractérisé en ce que** ladite étape d'analyse est réalisée à l'aide d'une unité de traitement dans laquelle sont mémorisés des signaux modélisés obtenus lors de ladite étape préalable.

11. Procédé selon la revendication 10, **caractérisé en ce qu'**il comprend une étape de comparaison du signal émis par le composé luminescent au travers de la coquille de l'oeuf pendant l'étape de détection avec les signaux modélisés obtenus lors de ladite étape préalable.

12. Procédé selon l'une quelconque des revendication précédentes, **caractérisé en ce que** ladite étape d'injection est conduite de façon à injecter simultanément ledit produit à injecter et ledit composé luminescent.

13. Procédé selon l'une quelconque des revendications précédentes, **caractérisé en ce que** ladite étape d'injection est conduite de façon à injecter séparément ledit produit à injecter et ledit composé luminescent.

14. Procédé selon les revendications 12 ou 13, **caractérisé en ce que** ledit composé luminescent est apte à venir se fixer sur le produit.

15. Procédé selon l'une quelconque des revendications 6 à 14, **caractérisé en ce que** ladite étape de soumission de l'oeuf à une source d'excitation lumineuse et ladite étape de détection du signal sont effectuées de part et d'autre de l'axe de symétrie dudit oeuf.

16. Procédé selon l'une quelconque des revendications 6 à 15, **caractérisé en ce que** lesdites étapes de soumission et de traitement de l'information recueillie sont effectuées pour au moins deux, préférentiellement au moins trois, positions angulaires distinctes relatives entre l'oeuf et ladite source d'excitation lumineuse.

17. Procédé selon l'une quelconque des revendications 6 à 16, **caractérisé en ce que** ladite étape de traitement de l'information recueillie n'est effectuée que sur la partie du signal émis par la partie supérieure de l'oeuf contenant la chambre à air.

## Patentansprüche

1. Verfahren zur Kontrolle einer Injektion eines Produkts zu Impfzwecken in ein Vogelei, das nacheinander die folgenden Schritte umfasst:
- Injektion einer Zusammensetzung in ein Ei, die ein Produkt umfasst, das eine Impfstoffaktivität aufweist und eine lumineszierende Verbindung, die einen Biomarker des Produkts bildet, das eine Impfstoffaktivität aufweist;
- Nachweis des von der lumineszierenden Verbindung abgegebenen Signals durch die Eischale hindurch;
- Verarbeitung der gesammelten Information.

2. Verfahren nach Anspruch 1, **dadurch gekennzeichnet, dass** die lumineszierende Verbindung eine fluoreszierende oder autolumineszierende oder chemolumineszierende Verbindung ist.

3. Verfahren nach einem der vorherigen Ansprüche, **dadurch gekennzeichnet, dass** die lumineszierende Verbindung Indocyaningrün oder dessen Derivate darstellt, wie etwa Salze, Ester oder mit Strukturelementen funktionalisierte Derivate.

4. Verfahren nach einem der vorherigen Ansprüche, **dadurch gekennzeichnet, dass** die lumineszierende Verbindung vom Typ S-B-A oder dessen Derivaten ist, wie etwa Salzen, Estern oder mit Strukturelementen funktionalisierten Derivaten.

5. Verfahren nach einem der vorherigen Ansprüche, **dadurch gekennzeichnet, dass** es einen Schritt der Korrelation des durch die lumineszierende Verbindung abgegebenen Signals und der Lokalisierung dieser im Ei umfasst.

6. Verfahren nach einem der vorherigen Ansprüche, **dadurch gekennzeichnet, dass** es einen Schritt des Aussetzens des Eis gegenüber einer Leuchtanregungsquelle umfasst, der zum Nachweis des Signals erforderlich ist.

7. Verfahren nach Anspruch 6, **dadurch gekennzeichnet, dass** die Leuchtanregungsquelle an die Wellenlängen der lumineszierenden Verbindung angepasst ist.

8. Verfahren nach den Ansprüchen 6 oder 7, **dadurch gekennzeichnet, dass** die Leuchtanregungsquelle ein Laser ist oder alle Filter, die an die Wellenlängen der lumineszierenden Verbindung angepasst sind.

9. Verfahren nach einem der vorherigen Ansprüche, **dadurch gekennzeichnet, dass** es einen vorausgehenden Schritt der Modellierung des durch die lumineszierende Verbindung abgegebenen Signals umfasst, wenn es sich in jeder der folgenden Zonen des Eis befindet:
- Luftkammer
- Allantoisflüssigkeit
- Amnionflüssigkeit
- Embryo.

10. Verfahren nach Anspruch 9, **dadurch gekennzeichnet, dass** der Analyseschritt mit Hilfe einer Verarbeitungseinheit durchgeführt wird, in der die beim vorherigen Schritt erhaltenen modellierten Signale abgespeichert sind.

11. Verfahren nach Anspruch 10, **dadurch gekennzeichnet, dass** es einen Schritt des Vergleichens des von der lumineszierenden Substanz durch die Eischale während des Nachweisschritts abgegebenen Signals mit den beim vorherigen Schritt erhaltenen modellierten Signalen umfasst.

12. Verfahren nach einem der vorherigen Ansprüche, **dadurch gekennzeichnet, dass** der Schritt der Injektion derart ausgeführt wird, dass das zu injizierende Produkt und die lumineszierende Verbindung gleichzeitig injiziert werden.

13. Verfahren nach einem der vorherigen Ansprüche, **dadurch gekennzeichnet, dass** der Schritt der Injektion derart ausgeführt wird, dass das zu injizierende Produkt und die lumineszierende Verbindung getrennt voneinander injiziert werden.

14. Verfahren nach den Ansprüchen 12 oder 13, **dadurch gekennzeichnet, dass** die lumineszierende Verbindung geeignet ist, sich an das Produkt zu binden.

15. Verfahren nach einem der Ansprüche 6 bis 14, **dadurch gekennzeichnet, dass** der Schritt des Aussetzens des Eis gegenüber einer Leuchtanregungsquelle und der Schritt des Nachweisens des Signals beidseitig der Symmetrieachse des Eis durchgeführt werden.

16. Verfahren nach einem der Ansprüche 6 bis 15, **dadurch gekennzeichnet, dass** die Schritte des Aussetzens und des Verabeitens der gesammelten Information für mindestens zwei, vorzugsweise mindestens drei unterschiedliche relative Winkelpositionen zwischen dem Ei und der Leuchtanregungsquelle durchgeführt werden.

17. Verfahren nach einem der Ansprüche 6 bis 16, **dadurch gekennzeichnet, dass** der Schritt des Verabeitens der gesammelten Information nur an dem Teilsignal durchgeführt wird, das durch den oberen Teil des Eis abgegeben wird, der die Luftkammer enthält.

## Claims

1. Method for testing an injection of a product for vaccine purposes in a bird egg, including, in sequence, the following steps:
- injection of a composition containing a product having a vaccine activity and a luminescent compound forming a biomarker of said product having a vaccine activity into an egg;
- detection of the signal emitted by the luminescent compound through the shell of the egg;
- processing of the information collected.

2. Method according to claim 1, **characterized in that** said luminescent compound is a fluorescent or autoluminescent or chemiluminescent compound.

3. Method according to any one of the preceding claims, **characterized in that** said luminescent compound is indocyanine green or derivatives thereof, such as salts, esters or derivatives functionalized with structural elements.

4. Method according to any one of the preceding claims, **characterized in that** said luminescent compound is of the S-B-A type or derivatives thereof, such as salts, esters or derivatives functionalized with structural elements.

5. Method according to any one of the preceding claims, **characterized in that** it includes a step of correlating the signal emitted by the luminescent compound and of locating it in the egg.

6. Method according to any one of the preceding claims, **characterized in that** it includes a step of subjecting the egg to a light excitation source necessary for detection of the signal.

7. Method according to claim 6, **characterized in that** the light excitation source is adapted to the wavelengths of the luminescent compound.

8. Method according to claim 6 or 7, **characterized in that** the excitation light source is a laser or any filter adapted to the wavelengths of the luminescent compound.

9. Method according to any one of the preceding claims, **characterized in that** it includes a preliminary step of modelling the signal emitted by the luminescent compound when it is located in each of the following areas of the egg:
- air chamber,
- allantoic fluid,
- amniotic fluid,
- embryo.

10. Method according to claim 9, **characterized in that** said analysis step is performed by means of a processing unit wherein modelled signals obtained in said previous step are stored.

11. Method according to claim 10, **characterized in that** it includes a step of comparing the signal emitted by the luminescent compound through the shell of the egg during the detection step with the modelled signals obtained in said previous step.

12. Method according to any one of the preceding claims, **characterized in that** said injection step is performed so as to simultaneously inject said product to be injected and said luminescent compound.

13. Method according any one of the preceding claims, **characterized in that** said injection step is performed so as to separately inject said product to be injected and said luminescent compound.

14. Method according to claims 12 or 13, **characterized in that** said luminescent compound is capable of binding to the product.

15. Method according to any one of claims 6 to 14, **characterized in that** said step of subjecting the egg to a light excitation source and said step of detecting the signal are performed on each side of the axis of symmetry of said egg.

16. Method according to any one of claims 6 to 15, **characterized in that** said steps of subjection and processing of the information collected are performed for at least two, preferably at least three, distinct relative angular positions between the egg and said light excitation source.

17. Method according to any one of claims 6 to 16, **characterized in that** said step of processing the information collected is performed only on the part of the signal emitted by the upper part of the egg containing the air chamber.
